# EUROPEAN PATENT APPLICATION

(11) **EP 4 636 019 A1**
(43) Date of publication of application: **22.10.2025**
(21) Application number: 23903428.3
(22) Date of filing: 07.12.2023
(51) Int. Cl.: C08J 11/10, C07C 29/09, C07C 31/12, C08F 8/12

(54) **METHOD FOR PRODUCING HYDROPHILIC POLYMER AND METHOD FOR PRODUCING ALCOHOL**

(30) Priority: 13.12.2022 JP 2022198281
(71) Applicant: NITTO DENKO CORPORATION, Ibaraki-shi Osaka 567-8680 (JP)
(72) Inventor: YAMADA, Yosuke, Ibaraki-shi, Osaka 567-8680 (JP); OJI, Katsunari, Ibaraki-shi, Osaka 567-8680 (JP); YAMANE, Toshihisa, Ibaraki-shi, Osaka 567-8680 (JP); KAWATAKE, Fumika, Ibaraki-shi, Osaka 567-8680 (JP); YAMAMOTO, Akiyoshi, Ibaraki-shi, Osaka 567-8680 (JP); KOTANI, Chihiro, Ibaraki-shi, Osaka 567-8680 (JP)
(74) Representative: Hoffmann Eitle
(86) International application number: PCT/JP2023/043924
(87) International publication number: WO 2024/128141

(57) **Abstract**

Provided are methods of producing, from an acrylic pressure-sensitive adhesive, a hydrophilic polymer serving as a recycled acrylic polymer that may serve as a raw material for a recycled pressure-sensitive adhesive, and an alcohol serving as a by-product of a hydrolysis reaction, without substantially using a treatment liquid such as a solvent. A method of producing a hydrophilic polymer according to an embodiment of the present invention is a method of producing a hydrophilic polymer from an acrylic pressure-sensitive adhesive, the method including a reaction step of subjecting the acrylic pressure-sensitive adhesive and an alkaline compound to a solid phase reaction. A method of producing an alcohol according to an embodiment of the present invention is a method of producing an alcohol from an acrylic pressure-sensitive adhesive, the method including a reaction step of subjecting the acrylic pressure-sensitive adhesive and an alkaline compound to a solid phase reaction.

## Description

### TECHNICAL FIELD

The present invention relates to a method of producing a hydrophilic polymer. The present invention also relates to a method of producing an alcohol.

### BACKGROUND ART

A pressure-sensitive adhesive tape has been used in a large amount in, for example, the bonding of a label or the like to an article or a wrapping material, the packaging of a packaging material, a production process for an electronic member or an optical member, and a masking application. In particular, in recent years, the frequency at which the pressure-sensitive adhesive tape is used in the production process for an electronic member or an optical member has been increasing, and hence a large amount of pressure-sensitive adhesive tape waste has been produced in, for example, a production site.

Typically, the pressure-sensitive adhesive tape waste is subjected to waste treatment by being burnt, or is subjected to waste treatment by being brought into a waste disposal site. However, such waste treatment is not preferred from the viewpoint of a reduction in environmental load, and from the viewpoint of resource circulation.

In view of the foregoing, such waste treatment for the pressure-sensitive adhesive tape waste as described above needs to be reduced to the extent possible, and the resources need to be reused. The recycling of a material for the pressure-sensitive adhesive tape is conceived as means for reducing such waste treatment for the pressure-sensitive adhesive tape. The establishment of a technology of recycling the material for the pressure-sensitive adhesive tape leads to, for example, the suppression of CO₂ emissions involved in fossil resource mining, and hence a large effect is expected to solve a problem of climate change.

Typical examples of the material for the pressure-sensitive adhesive tape include a pressure-sensitive adhesive and a base material. The pressure-sensitive adhesive tape is typically obtained by forming the pressure-sensitive adhesive on the base material as follows: a pressure-sensitive adhesive composition is formed by dissolving a polymer (sometimes referred to as "base polymer") and an additive such as a cross-linking agent depending on purposes in a solvent; the composition is applied to the base material; and heating, drying, or the like is performed as required to advance a cross-linking reaction (for example, Patent Literature 1).

### CITATION LIST

### Patent Literature

Patent Literature 1: JP 2021-175796 A

### SUMMARY OF INVENTION

### Technical Problem

An acrylic pressure-sensitive adhesive, which is a typical pressure-sensitive adhesive, has a cross-linked structure in which a main chain and a side chain are intricately entangled. Accordingly, the acrylic pressure-sensitive adhesive has poor handleability and processability when reused. In view of the foregoing, recently, the inventors of the present invention have made investigations on the production of a recycled acrylic polymer that may serve as a raw material for a recycled pressure-sensitive adhesive as a technology for reusing the acrylic pressure-sensitive adhesive. As one of the investigations, hydrolysis of the side chain with an alkaline compound has been investigated. In order to advance such hydrolysis, an entangled molecular chain needs to be relaxed to increase the probability of contact between a reaction site and the alkaline compound. In order to relax the molecular chain, a method involving using an organic solvent having high affinity with the acrylic pressure-sensitive adhesive, which is hydrophobic, to swell the pressure-sensitive adhesive is conceived. Meanwhile, the alkaline compound required for the hydrolysis is hydrophilic, and is hence insoluble in the organic solvent. In view of the foregoing, in order to swell the pressure-sensitive adhesive, it is important to select a treatment liquid that can dissolve the alkaline compound and that can be mixed with the organic solvent.

During investigations on the treatment liquid as described above, the inventors of the present invention have paid attention to the fact that a relatively large amount of the treatment liquid is required for swelling the pressure-sensitive adhesive. In addition, the inventors have considered that, when the amount of the treatment liquid increases, as well as the cost of the treatment liquid increases, the cost for achieving the recycling process for reuse increases by, for example, requiring a corresponding amount of energy in order to recover the used treatment liquid for a reduction in an environmental load. In addition, the inventors have also considered that a target product may remain in the recovered treatment liquid, which may cause a reduction in yield of the target product.

Thus, an object of the present invention is to provide methods of producing, from an acrylic pressure-sensitive adhesive, a hydrophilic polymer serving as a recycled acrylic polymer that may serve as a raw material for a recycled pressure-sensitive adhesive, and an alcohol serving as a by-product of a hydrolysis reaction, without substantially using a treatment liquid such as a solvent.

### Solution to Problem

The inventors of the present invention have made extensive investigations to solve the above-mentioned problems, and as a result, have conceived that a physical approach is adopted as technical means for relaxing a molecular chain. Then, the inventors have been able to solve the above-mentioned problem by subjecting an acrylic pressure-sensitive adhesive and an alkaline compound to a solid phase reaction, without substantially using a treatment liquid such as a solvent, by using, for example, a device capable of efficiently applying high shear force to the pressure-sensitive adhesive, such as a planetary mixer or a kneader.

In addition, the probability of contact between a reaction site of a hydrolysis reaction and the alkaline compound can be physically increased by such solid phase reaction. Accordingly, it is presumed that the alkaline compound is directly brought into contact with the reaction site of the hydrolysis reaction in a state of high concentration. As a result, it has also been recognized that an effect that a reaction time for the hydrolysis is dramatically shortened and an effect that the yield of a hydrophilic polymer serving as a recycled acrylic polymer to be obtained is high can be expressed. In addition, it has also been recognized that an effect that an alcohol produced together with the hydrophilic polymer is also obtained in a high yield can be expressed.

Further, an alcohol is also produced by the hydrolysis, and it has also been recognized that the following effect can be expressed by recovering the alcohol (preferably recovering the alcohol under reduced pressure): an alcohol having high purity is obtained with high efficiency. In addition, it has also been recognized that the following effect can be expressed by forcibly recovering the alcohol (preferably forcibly recovering the alcohol under reduced pressure): the hydrolysis reaction, which is an equilibrium reaction, becomes advantageous in a direction of hydrophilic polymer production by Le Chatelier's principle, and hence the yield of the hydrophilic polymer serving as the recycled acrylic polymer is further increased.
[1] According to one embodiment of the present invention, there is provided a method of producing a hydrophilic polymer from an acrylic pressure-sensitive adhesive, the method including a reaction step of subjecting the acrylic pressure-sensitive adhesive and an alkaline compound to a solid phase reaction.
[2] In the method of producing a hydrophilic polymer according to the above-mentioned item [1], the solid phase reaction may be performed by kneading the acrylic pressure-sensitive adhesive and the alkaline compound while applying shear force.
[3] In the method of producing a hydrophilic polymer according to the above-mentioned item [1] or [2], an alcohol may be generated through the reaction step.
[4] The method of producing a hydrophilic polymer according to any one of the above-mentioned items [1] to [3] may further include an alcohol recovery step of recovering the alcohol generated through the reaction step.
[5] In the method of producing a hydrophilic polymer according to the above-mentioned item [4], the alcohol recovery step may include recovering the alcohol generated through the reaction step under reduced pressure.
[6] The method of producing a hydrophilic polymer according to any one of the above-mentioned items [1] to [5] may further include an impurity separation step of separating an impurity.
[7] The method of producing a hydrophilic polymer according to any one of the above-mentioned items [1] to [6] may further include a drying step of drying the hydrophilic polymer.
[8] In the method of producing a hydrophilic polymer according to any one of the above-mentioned items [1] to [7], the acrylic pressure-sensitive adhesive may be an acrylic pressure-sensitive adhesive in a swollen gel state containing an organic solvent.
[9] In the method of producing a hydrophilic polymer according to the above-mentioned item [8], a content of the organic solvent in the acrylic pressure-sensitive adhesive in a swollen gel state may be 50 wt% or less.
[10] According to one embodiment of the present invention, there is provided a method of producing an alcohol from an acrylic pressure-sensitive adhesive, the method including a reaction step of subjecting the acrylic pressure-sensitive adhesive and an alkaline compound to a solid phase reaction.
[11] In the method of producing an alcohol according to the above-mentioned item [10], the solid phase reaction may be performed by kneading the acrylic pressure-sensitive adhesive and the alkaline compound while applying shear force.
[12] In the method of producing an alcohol according to any one of the above-mentioned item [10] or [11], the acrylic pressure-sensitive adhesive may be an acrylic pressure-sensitive adhesive in a swollen gel state containing an organic solvent.
[13] In the method of producing an alcohol according to the above-mentioned item [12], a content of the organic solvent in the acrylic pressure-sensitive adhesive in a swollen gel state may be 50 wt% or less.

### Advantageous Effects of Invention

According to one embodiment of the present invention, it is possible to provide the method of producing, from an acrylic pressure-sensitive adhesive, a hydrophilic polymer serving as a recycled acrylic polymer that may serve as a raw material for a recycled pressure-sensitive adhesive, and an alcohol serving as a by-product of a hydrolysis reaction, without substantially using a treatment liquid such as a solvent. In addition, according to one embodiment of the present invention, the probability of contact between a reaction site of a hydrolysis reaction and the alkaline compound can be physically increased. Accordingly, it is presumed that the alkaline compound is directly brought into contact with the reaction site of the hydrolysis reaction in a state of high concentration. As a result, an effect that a reaction time for the hydrolysis is dramatically shortened and an effect that a yield of a hydrophilic polymer to be obtained is high can be expressed. In addition, an effect that an alcohol produced together with the hydrophilic polymer is also obtained in a high yield can be expressed. Further, according to one embodiment of the present invention, an alcohol is also produced by the hydrolysis, and the following effect can be expressed by recovering the alcohol (preferably recovering the alcohol under reduced pressure): an alcohol having high purity is obtained with high efficiency. In addition, according to one embodiment of the present invention, the following effect can be expressed by forcibly recovering the alcohol produced by the hydrolysis (preferably forcibly recovering the alcohol under reduced pressure): the hydrolysis reaction, which is an equilibrium reaction, becomes advantageous in a direction of hydrophilic polymer production by Le Chatelier's principle, and hence the yield of the hydrophilic polymer serving as the recycled acrylic polymer is further increased.

### DESCRIPTION OF EMBODIMENTS

When the expression "weight" is used herein, the expression may be replaced with "mass" that has been commonly used as an SI unit for representing a weight.

When the expression "(meth)acryl" is used herein, the expression means "acryl and/or methacryl," when the expression "(meth)acrylate" is used herein, the expression means "acrylate and/or methacrylate," when the expression "(meth)allyl" is used herein, the expression means "allyl and/or methallyl," and when the expression "(meth)acrolein" is used herein, the expression means "acrolein and/or methacrolein."

### <<1. Method of producing Hydrophilic Polymer>>

A method of producing a hydrophilic polymer according to an embodiment of the present invention is a method of producing a hydrophilic polymer from an acrylic pressure-sensitive adhesive, the method including a reaction step of subjecting the acrylic pressure-sensitive adhesive and an alkaline compound to a solid phase reaction.

The acrylic pressure-sensitive adhesive is obtained by a cross-linking reaction of an acrylic pressure-sensitive adhesive composition containing an acrylic polymer serving as a base polymer and a cross-linking agent, and has a cross-linked structure in which a main chain and a side chain are intricately entangled. In the method of producing a hydrophilic polymer according to the embodiment of the present invention, cleavage of the cross-linked structure of a cross-linked polymer serving as a main polymer component in the acrylic pressure-sensitive adhesive and a hydrolysis reaction proceed extremely smoothly by performing the solid phase reaction between such acrylic pressure-sensitive adhesive and alkaline compound. As a result, a hydrophilic polymer having a carboxyl group on a side chain thereof can be produced without being significantly reduced in molecular weight as compared to the base polymer.

In addition, in the method of producing a hydrophilic polymer according to the embodiment of the present invention, the probability of contact between a reaction site of the hydrolysis reaction and the alkaline compound can be physically improved by adopting the solid phase reaction. Accordingly, it is presumed that the alkaline compound is directly brought into contact with the reaction site of the hydrolysis reaction in a state of high concentration. As a result, an effect that the reaction time for the hydrolysis is dramatically shortened and an effect that the yield of the hydrophilic polymer to be obtained is high can be expressed.

### <1-1. Acrylic Pressure-sensitive Adhesive>

Any appropriate acrylic pressure-sensitive adhesive may be adopted as the acrylic pressure-sensitive adhesive to be used in the method of producing a hydrophilic polymer according to the embodiment of the present invention to the extent that the effects of the present invention are not impaired. A typical example of such acrylic pressure-sensitive adhesive is an acrylic pressure-sensitive adhesive serving as waste (sometimes referred to as "waste acrylic pressure-sensitive adhesive"). Examples of the waste acrylic pressure-sensitive adhesive include: an acrylic pressure-sensitive adhesive in an acrylic pressure-sensitive adhesive tape recovered after its use; an acrylic pressure-sensitive adhesive present as an adhesive residue on an adherend having bonded thereto the acrylic pressure-sensitive adhesive tape at the time of the peeling of the acrylic pressure-sensitive adhesive tape from the adherend; an acrylic pressure-sensitive adhesive adhering to a production device for an acrylic pressure-sensitive adhesive; and an acrylic pressure-sensitive adhesive on an edge material portion, which does not become a product, of the acrylic pressure-sensitive adhesive tape after being cut into a predetermined product width.

The acrylic pressure-sensitive adhesive may be formed by any appropriate method. An example of such method is a method of forming the acrylic pressure-sensitive adhesive from an acrylic pressure-sensitive adhesive composition, more specifically, a method including: applying an acrylic pressure-sensitive adhesive composition onto any appropriate base material; heating and drying the composition as required; and curing the composition as required to form the pressure-sensitive adhesive (specifically, pressure-sensitive adhesive layer) on the base material. Examples of such application method include methods involving using a gravure roll coater, a reverse roll coater, a kiss roll coater, a dip roll coater, a bar coater, a knife coater, an air knife coater, a spray coater, a comma coater, a direct coater, a roll brush coater, and a die coater.

The acrylic pressure-sensitive adhesive composition preferably contains an acrylic polymer and a cross-linking agent because the effects of the present invention can be further expressed. The acrylic polymer is a polymer that may be called a base polymer in the field of acrylic pressure-sensitive adhesives. The number of kinds of the acrylic polymers may be only one, or two or more.

The content of the acrylic polymer in the acrylic pressure-sensitive adhesive composition is preferably from 50 wt% to 100 wt%, more preferably from 60 wt% to 100 wt%, still more preferably from 70 wt% to 100 wt%, particularly preferably from 80 wt% to 100 wt%, most preferably from 90 wt% to 100 wt% in terms of solid content.

Any appropriate acrylic polymer may be adopted as the acrylic polymer to the extent that the effects of the present invention are not impaired.

The weight-average molecular weight of the acrylic polymer is preferably from 100,000 to 3,000,000, more preferably from 150,000 to 2,000,000, still more preferably from 200,000 to 1,500,000, particularly preferably from 250,000 to 1,000,000. The glass transition temperature (Tg) of the acrylic polymer is preferably from -100°C to 30°C, more preferably from -95°C to 20°C, still more preferably from -90°C to 10°C, particularly preferably from -80°C to 0°C.

An example of the acrylic polymer is an acrylic polymer formed through polymerization from a composition (A) containing a (meth)acrylic acid alkyl ester whose alkyl ester moiety has an alkyl group having 1 to 12 carbon atoms (component (a)), and at least one kind selected from the group consisting of: a (meth)acrylic acid ester having a OH group; and (meth)acrylic acid (component (b)). The number of kinds of the components (a) and the number of kinds of the components (b) may each be independently only one, or two or more.

Examples of the (meth)acrylic acid alkyl ester whose alkyl ester moiety has an alkyl group having 1 to 12 carbon atoms (component (a)) include methyl (meth)acrylate, ethyl (meth)acrylate, propyl (meth)acrylate, n-butyl (meth)acrylate, isobutyl (meth)acrylate, s-butyl (meth)acrylate, t-butyl (meth)acrylate, pentyl (meth)acrylate, hexyl (meth)acrylate, heptyl (meth)acrylate, octyl (meth)acrylate, 2-ethylhexyl (meth)acrylate, isooctyl (meth)acrylate, nonyl (meth)acrylate, isononyl (meth)acrylate, decyl (meth)acrylate, isodecyl (meth)acrylate, undecyl (meth)acrylate, and dodecyl (meth)acrylate. Of those, n-butyl (meth)acrylate and 2-ethylhexyl (meth)acrylate are preferred, and n-butyl acrylate and 2-ethylhexyl acrylate are more preferred because the effects of the present invention can be further expressed.

Examples of the at least one kind selected from the group consisting of: a (meth)acrylic acid ester having a OH group; and (meth)acrylic acid (component (b)) include: (meth)acrylic acid esters each having a OH group, such as hydroxyethyl (meth)acrylate, hydroxypropyl (meth)acrylate, and hydroxybutyl (meth)acrylate; and (meth)acrylic acid. Of those, hydroxyethyl (meth)acrylate and (meth)acrylic acid are preferred, and hydroxyethyl acrylate and acrylic acid are more preferred because the effects of the present invention can be further expressed.

The composition (A) may contain a copolymerizable monomer except the component (a) and the component (b). The number of kinds of the copolymerizable monomers may be only one, or two or more. Examples of such copolymerizable monomer include: carboxyl group-containing monomers (provided that (meth)acrylic acid is excluded), such as itaconic acid, maleic acid, fumaric acid, crotonic acid, isocrotonic acid, and acid anhydrides thereof (e.g., acid anhydride group-containing monomers, such as maleic anhydride and itaconic anhydride); amide group-containing monomers, such as (meth)acrylamide, N,N-dimethyl (meth)acrylamide, N-methylol (meth)acrylamide, N-methoxymethyl (meth)acrylamide, N-butoxymethyl (meth)acrylamide, and N-hydroxyethyl (meth)acrylamide; amino group-containing monomers, such as aminoethyl (meth)acrylate, dimethylaminoethyl (meth)acrylate, and t-butylaminoethyl (meth)acrylate; epoxy group-containing monomers, such as glycidyl (meth)acrylate and methylglycidyl (meth)acrylate; cyano group-containing monomers, such as acrylonitrile and methacrylonitrile; heterocycle-containing vinyl-based monomers, such as N-vinyl-2-pyrrolidone, (meth)acryloylmorpholine, N-vinylpiperidone, N-vinylpiperazine, N-vinylpyrrole, N-vinylimidazole, vinylpyridine, vinylpyrimidine, and vinyloxazole; sulfonic acid group-containing monomers such as sodium vinylsulfonate; phosphoric acid group-containing monomers such as 2-hydroxyethylacryloyl phosphate; imide group-containing monomers, such as cyclohexylmaleimide and isopropylmaleimide; isocyanate group-containing monomers such as 2-methacryloyloxyethyl isocyanate; (meth)acrylic acid esters each having an alicyclic hydrocarbon group, such as cyclopentyl (meth)acrylate, cyclohexyl (meth)acrylate, and isobornyl (meth)acrylate; (meth)acrylic acid esters each having an aromatic hydrocarbon group, such as phenyl (meth)acrylate, phenoxyethyl (meth)acrylate, and benzyl (meth)acrylate; vinyl esters, such as vinyl acetate and vinyl propionate; aromatic vinyl compounds, such as styrene and vinyltoluene; olefins and dienes, such as ethylene, butadiene, isoprene, and isobutylene; vinyl ethers such as a vinyl alkyl ether; and vinyl chloride.

A polyfunctional monomer may also be adopted as the copolymerizable monomer. The term "polyfunctional monomer" refers to a monomer having two or more ethylenically unsaturated groups in a molecule thereof. Any appropriate ethylenically unsaturated groups may be adopted as the ethylenically unsaturated groups to the extent that the effects of the present invention are not impaired. Examples of such ethylenically unsaturated group include radically polymerizable functional groups, such as a vinyl group, a propenyl group, an isopropenyl group, a vinyl ether group (vinyloxy group), and an allyl ether group (allyloxy group). Examples of the polyfunctional monomer include hexanediol di(meth)acrylate, butanediol di(meth)acrylate, (poly)ethylene glycol di(meth)acrylate, (poly)propylene glycol di(meth)acrylate, neopentyl glycol di(meth)acrylate, pentaerythritol di(meth)acrylate, pentaerythritol tri(meth)acrylate, dipentaerythritol hexa(meth)acrylate, trimethylolpropane tri(meth)acrylate, tetramethylolmethane tri(meth)acrylate, allyl (meth)acrylate, vinyl (meth)acrylate, divinylbenzene, epoxy acrylate, polyester acrylate, and urethane acrylate. The number of kinds of such polyfunctional monomers may be only one, or two or more.

A (meth)acrylic acid alkoxyalkyl ester may also be adopted as the copolymerizable monomer. Examples of the (meth)acrylic acid alkoxyalkyl ester include 2-methoxyethyl (meth)acrylate, 2-ethoxyethyl (meth)acrylate, methoxytriethylene glycol (meth)acrylate, 3-methoxypropyl (meth)acrylate, 3-ethoxypropyl (meth)acrylate, 4-methoxybutyl (meth)acrylate, and 4-ethoxybutyl (meth)acrylate. The number of kinds of the (meth)acrylic acid alkoxyalkyl esters may be only one, or two or more.

The content of the (meth)acrylic acid alkyl ester whose alkyl ester moiety has an alkyl group having 1 to 12 carbon atoms (component (a)) is preferably 50 wt% or more, more preferably from 60 wt% to 100 wt%, still more preferably from 70 wt% to 100 wt%, particularly preferably from 80 wt% to 100 wt% with respect to the total amount (100 wt%) of the monomer components for forming the acrylic polymer because the effects of the present invention can be further expressed.

The content of the at least one kind selected from the group consisting of: a (meth)acrylic acid ester having a OH group; and (meth)acrylic acid (component (b)) is preferably 0.1 wt% or more, more preferably from 1.0 wt% to 50 wt%, still more preferably from 1.5 wt% to 40 wt%, particularly preferably from 2.0 wt% to 30 wt% with respect to the total amount (100 wt%) of the monomer components for forming the acrylic polymer because the effects of the present invention can be further expressed.

The composition (A) may contain any appropriate other component to the extent that the effects of the present invention are not impaired. Examples of such other component include a polymerization initiator, a chain transfer agent, and a solvent. Any appropriate content may be adopted as the content of each of those other components to the extent that the effects of the present invention are not impaired. The number of kinds of such other components may be only one, or two or more.

The acrylic pressure-sensitive adhesive composition may contain a cross-linking agent. When the cross-linking agent is used, the cohesive strength of the acrylic pressure-sensitive adhesive can be improved, and hence the effects of the present invention can be further expressed. The number of kinds of the cross-linking agents may be only one, or two or more.

Examples of the cross-linking agent include a polyfunctional isocyanate-based cross-linking agent, an epoxy-based cross-linking agent, a melamine-based cross-linking agent, and a peroxide-based cross-linking agent, and as well, a urea-based cross-linking agent, a metal alkoxide-based cross-linking agent, a metal chelate-based cross-linking agent, a metal salt-based cross-linking agent, a carbodiimide-based cross-linking agent, an oxazoline-based cross-linking agent, an aziridine-based cross-linking agent, and an amine-based cross-linking agent. Of those, at least one kind selected from the group consisting of: a polyfunctional isocyanate-based cross-linking agent; and an epoxy-based cross-linking agent is preferred because the effects of the present invention can be further expressed.

Examples of the polyfunctional isocyanate-based cross-linking agent include: lower aliphatic polyisocyanates, such as 1,2-ethylene diisocyanate, 1,4-butylene diisocyanate, and 1,6-hexamethylene diisocyanate; alicyclic polyisocyanates, such as cyclopentylene diisocyanate, cyclohexylene diisocyanate, isophorone diisocyanate, hydrogenated tolylene diisocyanate, and hydrogenated xylene diisocyanate; and aromatic polyisocyanates, such as 2,4-tolylene diisocyanate, 2,6-tolylene diisocyanate, 4,4'-diphenylmethane diisocyanate, and xylylene diisocyanate. Examples of the polyfunctional isocyanate-based cross-linking agent also include commercially available products, such as a trimethylolpropane/tolylene diisocyanate adduct (manufactured by Mitsui Chemicals, Inc., product name: "TAKENATE D101A"), a trimethylolpropane/hexamethylene diisocyanate adduct (manufactured by Tosoh Corporation, product name: "CORONATE HL", product name: "CORONATE HX"), and a trimethylolpropane/xylylene diisocyanate adduct (manufactured by Mitsui Chemicals, Inc., product name: "TAKENATE 110N").

Examples of the epoxy-based cross-linking agent (polyfunctional epoxy compound) include N,N,N',N'-tetraglycidyl-m-xylenediamine, diglycidylaniline, 1,3-bis(N,N-diglycidylaminomethyl)cyclohexane, 1,6-hexanediol diglycidyl ether, neopentyl glycol diglycidyl ether, ethylene glycol diglycidyl ether, propylene glycol diglycidyl ether, polyethylene glycol diglycidyl ether, polypropylene glycol diglycidyl ether, sorbitol polyglycidyl ether, glycerol polyglycidyl ether, pentaerythritol polyglycidyl ether, polyglycerol polyglycidyl ether, sorbitan polyglycidyl ether, trimethylolpropane polyglycidyl ether, adipic acid diglycidyl ester, o-phthalic acid diglycidyl ester, triglycidyl-tris(2-hydroxyethyl) isocyanurate, resorcinol diglycidyl ether, bisphenol-S-diglycidyl ether, and an epoxy-based resin having two or more epoxy groups in a molecule thereof. Examples of the epoxy-based cross-linking agent also include commercially available products such as a product available under the product name "TETRAD-C" (manufactured by Mitsubishi Gas Chemical Company, Inc.).

Any appropriate content may be adopted as the content of the cross-linking agent in the acrylic pressure-sensitive adhesive composition to the extent that the effects of the present invention are not impaired. Such content is, for example, preferably from 0.1 part by weight to 20 parts by weight, more preferably from 0.3 part by weight to 10 parts by weight, still more preferably from 0.5 part by weight to 8.0 parts by weight, particularly preferably from 1.0 part by weight to 6.0 parts by weight with respect to the solid content (100 parts by weight) of the acrylic polymer.

The acrylic pressure-sensitive adhesive composition may contain any appropriate other component to the extent that the effects of the present invention are not impaired. Examples of such other component include a polymer component except the acrylic polymer, a cross-linking accelerator, a cross-linking catalyst, a silane coupling agent, a tackifier resin (e.g., a rosin derivative, a polyterpene resin, a petroleum resin, or an oil-soluble phenol), an age resistor, an inorganic filler, an organic filler, a metal powder, a colorant (e.g., a pigment or a dye), a foil-like material, a UV absorber, an antioxidant, a light stabilizer, a chain transfer agent, a plasticizer, a softening agent, a surfactant, an antistatic agent, a conductive agent, a stabilizer, a surface lubricant, a leveling agent, a corrosion inhibitor, a heat stabilizer, a polymerization inhibitor, a lubricant, a solvent, and a catalyst.

### <1-2. Alkaline Compound>

Any appropriate solid alkaline compound may be adopted as the alkaline compound to the extent that the effects of the present invention are not impaired. Examples of such alkaline compound include: hydroxides or carbonates of alkali metals or alkaline earth metals, such as potassium hydroxide, sodium hydroxide, and calcium hydroxide; and metal alkoxides, such as sodium methoxide, sodium ethoxide, and potassium t-butoxide. Of those, at least one kind selected from the group consisting of: potassium hydroxide; sodium hydroxide, and sodium ethoxide is preferred.

### <1-3. Reaction Step>

In the reaction step, the acrylic pressure-sensitive adhesive and the alkaline compound are subjected to the solid phase reaction.

In theory, the usage amount of the alkaline compound with respect to the acrylic pressure-sensitive adhesive only needs to be set to an amount required for the hydrolysis of the acrylic pressure-sensitive adhesive. When a large amount of an unreacted alkaline compound is present in a product, post-treatment for treating the alkaline compound is required. In order to maximize recovery amounts of the hydrophilic polymer and the alcohol, a typical usage amount of the alkaline compound with respect to the acrylic pressure-sensitive adhesive is preferably from 0.5 molar equivalent to 1.2 molar equivalent, more preferably from 0.8 molar equivalent to 1.1 molar equivalent, still more preferably from 0.9 molar equivalent to 1.1 molar equivalent with respect to 1 molar equivalent of an acrylic pressure-sensitive adhesive base polymer.

The solid phase reaction is a reaction in a solid state including subjecting the acrylic pressure-sensitive adhesive and the alkaline compound (typically, a solid alkaline compound) to a reaction, without substantially using a liquid such as a solvent. The term "without substantially using" as used herein means being free from being intentionally added from outside for the purpose of being involved in the reaction, and unintentional and unavoidable incorporation of the liquid into the reaction system such as incorporation of water from moisture from a reaction atmosphere is included in the above-mentioned scope "without substantially using."

With regard to the acrylic pressure-sensitive adhesive to be used in the method of producing a hydrophilic polymer according to the embodiment of the present invention, a liquid such as a solvent that may be originally contained in the acrylic pressure-sensitive adhesive to the extent that the solid state of the acrylic pressure-sensitive adhesive is not inhibited is understood to fall outside the above-mentioned scope "substantially using a treatment liquid such as a solvent."

In addition, the addition of an organic solvent to the acrylic pressure-sensitive adhesive in order to adjust the softness of the acrylic pressure-sensitive adhesive is also included in the above-mentioned scope "without substantially using." In this case, the acrylic pressure-sensitive adhesive to be subjected to the solid phase reaction with the alkaline compound in the solid phase reaction is an acrylic pressure-sensitive adhesive in a swollen gel state containing the organic solvent. Any appropriate content may be adopted as the content of the organic solvent in the acrylic pressure-sensitive adhesive in a swollen gel state as long as the swollen gel state can be expressed. Such content of the organic solvent in the acrylic pressure-sensitive adhesive in a swollen gel state is preferably 50 wt% or less. Herein, the content of the organic solvent in the acrylic pressure-sensitive adhesive in a swollen gel state may be regarded as the same ratio as the ratio of the amount of the organic solvent to be added with respect to the total amount of the acrylic pressure-sensitive adhesive before the addition of the organic solvent and the organic solvent to be added.

Any appropriate organic solvent may be adopted as the organic solvent that may be added to the acrylic pressure-sensitive adhesive to the extent that the effects of the present invention are not impaired. The number of kinds of the organic solvents may be only one, or two or more. Such organic solvent is preferably an alcohol. Any appropriate alcohol may be adopted as the alcohol to the extent that the effects of the present invention are not impaired. Examples of such alcohol include methanol, ethanol, 1-propanol, 2-propanol, 1-butanol, 1-pentanol, 1-hexanol, cyclohexanol, benzyl alcohol, 1-heptanol, 1-octanol, and 2-ethylhexanol. In addition, an organic solvent except the alcohol may also be used as the organic solvent. For example, a solvent having high affinity with the acrylic pressure-sensitive adhesive, such as a hydrocarbon-based solvent (e.g., hexane, heptane, octane, nonane, decane, toluene, or methylcyclohexane), a ketone-based solvent (e.g., acetone or methyl ethyl ketone), or an ether-based solvent (e.g., diethyl ether or cyclopentyl methyl ether), may be selected as such organic solvent. Of course, the organic solvent that may be added to the acrylic pressure-sensitive adhesive is not limited thereto.

As described above, a typical example of the acrylic pressure-sensitive adhesive is an acrylic pressure-sensitive adhesive serving as waste (sometimes referred to as "waste acrylic pressure-sensitive adhesive"). Examples of the waste acrylic pressure-sensitive adhesive include: an acrylic pressure-sensitive adhesive in an acrylic pressure-sensitive adhesive tape recovered after its use; an acrylic pressure-sensitive adhesive present as an adhesive residue on an adherend having bonded thereto the acrylic pressure-sensitive adhesive tape at the time of the peeling of the acrylic pressure-sensitive adhesive tape from the adherend; and an acrylic pressure-sensitive adhesive adhering to a production device for an acrylic pressure-sensitive adhesive. The method of producing a hydrophilic polymer according to the embodiment of the present invention includes using such acrylic pressure-sensitive adhesive as it is, without substantially using a liquid such as a solvent.

The solid phase reaction may be performed by any appropriate method to the extent that the effects of the present invention are not impaired as long as the method can subject the acrylic pressure-sensitive adhesive and the alkaline compound to the reaction in a solid state, without substantially using a liquid such as a solvent. The solid phase reaction is preferably performed by kneading the acrylic pressure-sensitive adhesive and the alkaline compound while applying shear force because the effects of the present invention can be further expressed.

One preferred selection index for means for kneading while applying shear force is, for example, using a kneading device capable of applying a shear force [Pa] equivalent to the storage modulus of elasticity [Pa] (typically, 10³ Pa to 10⁹ Pa) of the acrylic pressure-sensitive adhesive. Examples of such kneading device include a planetary mixer, a kneader, KNEADEX, a single-screw extruder, and a twin-screw extruder.

Another preferred selection index for the means for kneading while applying shear force is, for example, using a kneading device having a high stirring intensity [kW/m³]. Such stirring intensity [kW/m³] is preferably 25 kW/m³ or more, more preferably 40 kW/m³ or more, still more preferably 60 kW/m³ or more, particularly preferably 80 kW/m³ or more. Examples of the kneading device having such stirring intensity include a planetary mixer, a kneader, KNEADEX, a single-screw extruder, and a twin-screw extruder.

The stirring intensity is also calculated from a ratio of a rated current value [A] of the device to a material capacity [L]. Such stirring intensity is preferably 0.9 A/L or more, more preferably 1.0 A/L or more, still more preferably 1.1 A/L or more, particularly preferably 1.2 A/L or more. Examples of the kneading device having such stirring intensity include a planetary mixer, a kneader, KNEADEX, a single-screw extruder, and a twin-screw extruder.

In the solid phase reaction, the kneading is preferably performed by loading the acrylic pressure-sensitive adhesive and the alkaline compound into the kneading device as described above. As the order of loading the acrylic pressure-sensitive adhesive and the alkaline compound into the kneading device, any one thereof may be loaded first, or both may be loaded simultaneously. Because the effects of the present invention can be further expressed, the order of loading the acrylic pressure-sensitive adhesive and the alkaline compound to the kneading device is preferably as follows: the acrylic pressure-sensitive adhesive is loaded into the kneading device, and then the alkaline compound is loaded into the kneading device, and is more preferably as follows: the acrylic pressure-sensitive adhesive is loaded into the kneading device and stirred in advance, and then the alkaline compound is loaded into the kneading device. When the loading is performed as described above, the probability of contact between the reaction site of the hydrolysis reaction and the alkaline compound can be physically further improved, and the effects of the present invention can be further expressed. Any appropriate time may be set for the time for stirring in advance to the extent that the effects of the present invention are not impaired. The time for such stirring in advance is preferably from 10 seconds to 30 minutes, more preferably from 30 seconds to 20 minutes, still more preferably from 1 minute to 10 minutes.

The alkaline compound may be pulverized in advance before being loaded into the kneading device.

When the solid phase reaction is advanced, the hydrolysis reaction is advanced in the reaction system (typically, in the kneading device), and a solid mixture containing the hydrophilic polymer is generated as well as an alcohol is generated as the liquid or a vapor thereof.

As described above, in the reaction step, the hydrolysis reaction is advanced in the reaction system (typically, in the kneading device) by the solid phase reaction between the acrylic pressure-sensitive adhesive and the alkaline compound, and the solid mixture containing the hydrophilic polymer is obtained as well as the alcohol is generated as the liquid or the vapor thereof.

### <1-4. Alcohol Recovery Step>

The method of producing a hydrophilic polymer according to the embodiment of the present invention may include an alcohol recovery step of recovering the alcohol generated through the reaction step.

In the alcohol recovery step, the alcohol generated through the reaction step is recovered. Any appropriate method may be adopted as a method of recovering the alcohol to the extent that the effects of the present invention are not impaired. An example of such method is recovery under reduced pressure because the effects of the present invention can be further expressed. Such method for recovery under reduced pressure includes, for example, reducing a pressure in the reaction system (typically, in the kneading device) to trap and recover the alcohol generated in the reaction system (typically, in the kneading device).

When the alcohol recovery step is adopted, the alcohol can be forcibly removed from the reaction system (typically, in the kneading device). Thus, the hydrolysis reaction, which is an equilibrium reaction, becomes advantageous in a direction of hydrophilic polymer production by Le Chatelier's principle, and hence an effect that the yield of the hydrophilic polymer is further increased can be expressed.

### <1-5. Impurity Separation Step>

The method of producing a hydrophilic polymer according to the embodiment of the present invention may include an impurity separation step of separating an impurity.

In the impurity separation step, an impurity from the solid mixture containing the hydrophilic polymer obtained through the reaction step is separated. Any appropriate method may be adopted as a method of separating an impurity from the solid mixture containing the hydrophilic polymer to the extent that the effects of the present invention are not impaired. Because the effects of the present invention can be further expressed, examples of such method include: (1) a method including dissolving the solid mixture containing the hydrophilic polymer in any appropriate liquid (typically, water), neutralizing the remaining alkaline compound with an acid (e.g., acetic acid) as required, and then separating an impurity by centrifugation; and (2) a method including adding any appropriate washing liquid (e.g., a mixed solvent of ethyl acetate and methanol) to the solid mixture containing the hydrophilic polymer, and separating an impurity by washing.

The impurity is separated from the solid mixture containing the hydrophilic polymer through the impurity separation step. Thus, a hydrophilic polymer is obtained.

### <1-6. Drying Step>

The method of producing a hydrophilic polymer according to the embodiment of the present invention may include a drying step of drying the hydrophilic polymer.

In the drying step, the hydrophilic polymer obtained through the impurity separation step is dried. Any appropriate conditions may be adopted as drying conditions to the extent that the effects of the present invention are not impaired. Examples of such conditions include a drying temperature of from 80°C to 150°C and a drying time of from 0.5 hour to 20 hours.

### <1-7. Other Step>

The method of producing a hydrophilic polymer according to the embodiment of the present invention may include any appropriate other step to the extent that the effects of the present invention are not impaired.

### <<2. Method of producing Alcohol>>

In the method of producing a hydrophilic polymer according to the embodiment of the present invention, the alcohol is generated by the hydrolysis reaction as described above. Accordingly, the method of producing a hydrophilic polymer according to the embodiment of the present invention may be regarded as a method of producing an alcohol from a different viewpoint. That is, a method of producing an alcohol according to an embodiment of the present invention is a method of producing an alcohol from an acrylic pressure-sensitive adhesive, the method including a reaction step of subjecting the acrylic pressure-sensitive adhesive and an alkaline compound to a solid phase reaction.

The acrylic pressure-sensitive adhesive is obtained by a cross-linking reaction of an acrylic pressure-sensitive adhesive composition containing an acrylic polymer serving as a base polymer and a cross-linking agent, and has a cross-linked structure in which a main chain and a side chain are intricately entangled. In the method of producing an alcohol according to the embodiment of the present invention, cleavage of the cross-linked structure of a cross-linked polymer serving as a main polymer component in the acrylic pressure-sensitive adhesive and a hydrolysis reaction proceed extremely smoothly by performing the solid phase reaction between such acrylic pressure-sensitive adhesive and alkaline compound. As a result, an alcohol can be produced.

In addition, in the method of producing an alcohol according to the embodiment of the present invention, the probability of contact between a reaction site of the hydrolysis reaction and the alkaline compound can be physically improved by adopting the solid phase reaction. Accordingly, it is presumed that the alkaline compound is directly brought into contact with the reaction site of the hydrolysis reaction in a state of high concentration. As a result, an effect that the reaction time for the hydrolysis is dramatically shortened and an effect that the yield of the alcohol to be generated is high can be expressed.

For the acrylic pressure-sensitive adhesive to be used in the method of producing an alcohol according to the embodiment of the present invention, reference may be made to the description in the section <1-1. Acrylic Pressure-sensitive Adhesive>.

For the alkaline compound to be used in the method of producing an alcohol according to the embodiment of the present invention, reference may be made to the description in the section <1-2. Alkaline Compound>.

For the reaction step in the method of producing an alcohol according to the embodiment of the present invention, reference may be made to the description in the section <1-3. Reaction Step>.

The method of producing an alcohol according to the embodiment of the present invention may include an alcohol recovery step of recovering the alcohol generated through the reaction step. For such alcohol recovery step, reference may be made to the description in the section <1-4. Alcohol Recovery Step>.

The method of producing an alcohol according to the embodiment of the present invention may include any appropriate other step to the extent that the effects of the present invention are not impaired.

### EXAMPLES

The present invention is specifically described below by way of Examples. However, the present invention is by no means limited to these Examples. Test and evaluation methods in Examples and the like are as described below.

### <Calculation of Carboxylic Acid Conversion Rate>

A carboxylic acid conversion rate was calculated from a ratio between the peak intensities of (meth)acrylic acid and a (meth)acrylic acid ester in a product in ¹³C-NMR.

### [Production Example 1]

As a model of the acrylic pressure-sensitive adhesive to be used in the method of producing a hydrophilic polymer and the method of producing an alcohol according to the embodiments of the present invention, an acrylic pressure-sensitive adhesive (A) was produced as described below.

A four-necked flask with a stirring blade, a temperature gauge, a nitrogen gas inlet tube, and a condenser was loaded with 95 parts by weight of butyl acrylate (BA) and 5 parts by weight of acrylic acid (AA) serving as monomers, 0.2 part by weight of 2,2'-azobisisobutyronitrile serving as a polymerization initiator, and 233 parts by weight of ethyl acetate. While the contents were gently stirred, a nitrogen gas was introduced, and the contents were heated to 60°C. While the liquid temperature in the flask was kept at around 60°C, a polymerization reaction was performed for 7 hours to provide a solution of an acrylic polymer (a) having a weight-average molecular weight of 600000.

100 Parts by weight (solid content) of the acrylic polymer (a), 5 parts by weight of an isocyanate compound (TAKENATE D101A, manufactured by Mitsui Chemicals, Inc.) serving as a cross-linking agent, and 0.03 part by weight of EMBILIZER OL-1 (manufactured by Tokyo Fine Chemical Co., Ltd.) serving as a catalyst were blended, and the blend was diluted with ethyl acetate so that an entire solid content became 20 wt% to provide an acrylic pressure-sensitive adhesive composition.

The resultant acrylic pressure-sensitive adhesive composition was applied to a polyethylene terephthalate film (product name: "T100", thickness: 50 µm, manufactured by Mitsubishi Chemical Corporation) so as to have a thickness of 10 µm after drying, and was cured and dried under the conditions of a drying temperature of 130°C and a drying time of 30 seconds. Thus, the acrylic pressure-sensitive adhesive (A) was produced.

### [Production Example 2]

As a model of the acrylic pressure-sensitive adhesive to be used in the method of producing a hydrophilic polymer and the method of producing an alcohol according to the embodiments of the present invention, an acrylic pressure-sensitive adhesive (B) was produced as described below.

A four-necked flask with a stirring blade, a temperature gauge, a nitrogen gas inlet tube, and a condenser was loaded with 64 parts by weight of ethyl acrylate (EA), 28 parts by weight of 2-ethylhexyl acrylate (2EHA), 5 parts by weight of methyl methacrylate (MMA), and 4 parts by weight of hydroxyethyl acrylate (HEA) serving as monomers, 0.2 part by weight of benzoyl peroxide serving as a polymerization initiator, and 233 parts by weight of ethyl acetate. While the contents were gently stirred, a nitrogen gas was introduced, and the contents were heated to 60°C. While the liquid temperature in the flask was kept at around 60°C, a polymerization reaction was performed for 7 hours to provide a solution of an acrylic polymer (b) having a weight-average molecular weight of 460000.

100 Parts by weight (solid content) of the acrylic polymer (b), 1.45 parts by weight of an isocyanate compound (TAKENATE D101A, manufactured by Mitsui Chemicals, Inc.) serving as a cross-linking agent, and 0.053 part by weight of EMBILIZER OL-1 (manufactured by Tokyo Fine Chemical Co., Ltd.) serving as a catalyst were blended, and the blend was diluted with ethyl acetate so that an entire solid content became 20 wt% to provide an acrylic pressure-sensitive adhesive composition.

The resultant acrylic pressure-sensitive adhesive composition was applied to a polyethylene terephthalate film (product name: "Diafoil MRF", thickness: 38 µm, manufactured by Mitsubishi Chemical Corporation) subjected to peeling treatment so as to have a thickness of 10 µm after drying, and was cured and dried under the conditions of a drying temperature of 130°C and a drying time of 30 seconds. Thus, the acrylic pressure-sensitive adhesive (B) was produced.

### [Example 1]

100 g of the acrylic pressure-sensitive adhesive (A) obtained in Production Example 1 was prepared, loaded into a planetary mixer (HIVIS DISPER MIX Model 3D-2, manufactured by Primix Corporation) serving as a reaction device, and subjected to stirring in advance for 5 minutes.

Subsequently, 54 g of pulverized potassium hydroxide (KOH, purity: 85%) was loaded, and stirring was continued for 0.5 hour while a stirring rotation speed was increased.

When the reaction was advanced, vapor and a liquid were generated in the reaction device, and the generated vapor and liquid were recovered in a trap can cooled with chiller water at 5°C under reduced pressure by reducing a pressure in the reaction device. The weight of the recovered liquid was 50 g. The structure of a main component of the recovered liquid obtained by the recovery under reduced pressure was identified by NMR (AVANCE III-600 with Cryo Probe, manufactured by Bruker Biospin). As a result, the main component was able to be identified as 1-butanol. According to GC/MS analysis, 1-butanol and BA serving as a raw material were present in a liquid derived from the polymer out of the recovered liquid, and the purity of 1-butanol was 97%.

After the completion of the reaction, a solid substance remained in the reaction device. Water was added to the solid substance to dissolve the solid substance, and acetic acid was added to perform neutralization. The resultant was then subjected to a centrifuge so that an impurity was centrifuged, and the supernatant was dried at 130°C for 2 hours. As a result, 85 g of potassium polyacrylate serving as a hydrophilic polymer was obtained. The structure of the resultant potassium polyacrylate was analyzed by NMR (AVANCE III-600 with Cryo Probe, manufactured by Bruker Biospin).

The results are shown in Table 1.

### [Example 2]

3.5 kg of the acrylic pressure-sensitive adhesive (A) obtained in Production Example 1 was prepared, loaded into a double arm kneader (Model TK5-2, manufactured by Toshin Co., Ltd.) serving as a reaction device, and subjected to stirring in advance for 5 minutes.

Subsequently, 1.87 kg of pulverized potassium hydroxide (KOH, purity: 85%) was loaded, and stirring was continued for 0.5 hour while a stirring rotation speed was increased.

When the reaction was advanced, vapor and a liquid were generated in the reaction device, and the generated vapor and liquid were recovered in a trap can cooled with chiller water at 5°C under reduced pressure by reducing a pressure in the reaction device. The weight of the recovered liquid was 1.7 kg. The structure of a main component of the recovered liquid obtained by the recovery under reduced pressure was identified by **NMR** (AVANCE III-600 with Cryo Probe, manufactured by Bruker Biospin). As a result, the main component was able to be identified as 1-butanol. According to GC/MS analysis, 1-butanol and BA serving as a raw material were present in a liquid derived from the polymer out of the recovered liquid, and the purity of 1-butanol was 97%.

After the completion of the reaction, a solid substance remained in the reaction device. The solid substance was loaded into a pressure kneader and pulverized, and a solvent containing ethyl acetate and methanol at a volume ratio of 1/1 was added thereto. The resultant was stirred to be washed, and the resultant solid substance was dried at 130°C for 2 hours. As a result, 3 kg of potassium polyacrylate serving as a hydrophilic polymer was obtained. The structure of the resultant potassium polyacrylate was analyzed by NMR (AVANCE III-600 with Cryo Probe, manufactured by Bruker Biospin).

The results are shown in Table 1.

### [Example 3]

3.5 kg of the acrylic pressure-sensitive adhesive (A) obtained in Production Example 1 was prepared, loaded into Trimix (manufactured by Inoue Mfg., Inc., corresponding to a planetary mixer) serving as a reaction device, and subjected to stirring in advance for 5 minutes.

Subsequently, 1.17 kg of pulverized sodium hydroxide (NaOH, purity: 97%) was loaded, and stirring was continued for 0.9 hour while a stirring rotation speed was increased.

When the reaction was advanced, vapor and a liquid were generated in the reaction device, and the generated vapor and liquid were recovered in a trap can cooled with chiller water at 5°C under reduced pressure by reducing a pressure in the reaction device. The weight of the recovered liquid was 1.7 kg. The structure of a main component of the recovered liquid obtained by the recovery under reduced pressure was identified by NMR (AVANCE III-600 with Cryo Probe, manufactured by Bruker Biospin). As a result, the main component was able to be identified as 1-butanol. According to GC/MS analysis, 1-butanol and BA serving as a raw material were present in a liquid derived from the polymer out of the recovered liquid, and the purity of 1-butanol was 97%.

After the completion of the reaction, a solid substance remained in the reaction device. The solid substance was pulverized in Trimix as it was, and a solvent containing ethyl acetate and methanol at a volume ratio of 1/1 was added thereto. The resultant was stirred to be washed, and the resultant solid substance was dried at 130°C for 2 hours. As a result, 2.5 kg of sodium polyacrylate serving as a hydrophilic polymer was obtained. The structure of the resultant sodium polyacrylate was analyzed by NMR (AVANCE III-600 with Cryo Probe, manufactured by Bruker Biospin).

The results are shown in Table 1.

### [Example 4]

3.5 kg of the acrylic pressure-sensitive adhesive (A) obtained in Production Example 1 was prepared, loaded into Trimix (manufactured by Inoue Mfg., Inc., corresponding to a planetary mixer) serving as a reaction device, and subjected to stirring in advance for 5 minutes.

Subsequently, 1.87 kg of pulverized potassium hydroxide (KOH, purity: 85%) was loaded, and stirring was continued for 0.5 hour while a stirring rotation speed was increased.

When the reaction was advanced, vapor and a liquid were generated in the reaction device, and the generated vapor and liquid were recovered in a trap can cooled with chiller water at 5°C under reduced pressure by reducing a pressure in the reaction device. The weight of the recovered liquid was 1.7 kg. The structure of a main component of the recovered liquid obtained by the recovery under reduced pressure was identified by NMR (AVANCE III-600 with Cryo Probe, manufactured by Bruker Biospin). As a result, the main component was able to be identified as 1-butanol. According to GC/MS analysis, 1-butanol and BA serving as a raw material were present in a liquid derived from the polymer out of the recovered liquid, and the purity of 1-butanol was 97%.

After the completion of the reaction, a solid substance remained in the reaction device. The solid substance was pulverized in Trimix as it was, and a solvent containing ethyl acetate and methanol at a volume ratio of 1/1 was added thereto. The resultant was stirred to be washed, and the resultant solid substance was dried at 130°C for 2 hours. As a result, 3 kg of potassium polyacrylate serving as a hydrophilic polymer was obtained. The structure of the resultant potassium polyacrylate was analyzed by NMR (AVANCE III-600 with Cryo Probe, manufactured by Bruker Biospin).

The results are shown in Table 1.

### [Example 5]

3 kg of the acrylic pressure-sensitive adhesive (B) obtained in Production Example 2 was prepared, loaded into Trimix (TX-15, manufactured by Inoue Mfg., Inc.) serving as a reaction device, and subjected to stirring in advance for 5 minutes.

Subsequently, 1.6 kg of pulverized potassium hydroxide (KOH, purity: 85%) was loaded, and stirring was continued for 5 hours while a stirring rotation speed was increased.

When the reaction was advanced, vapor and a liquid were generated in the reaction device, and the generated vapor and liquid were recovered in a solvent recovery device (CA103, manufactured by Kobex Co., Ltd.) cooled with chiller water at 15°C under reduced pressure by reducing a pressure in the reaction device. The weight of the recovered liquid was 0.1 kg. The structures of the components derived from the pressure-sensitive adhesive of the recovered liquid obtained by the recovery under reduced pressure were identified by GC/MS analysis. As a result, the components were able to be identified as ethanol and 2-ethylhexanol.

After the completion of the reaction, a solid substance remained in the reaction device. The solid substance was pulverized with a pulverizer (Forcemill FM1), and a solvent containing ethyl acetate and methanol at a volume ratio of 1/1 was added thereto. The resultant was stirred to be washed, and the resultant solid substance was dried at 130°C for 2 hours. As a result, potassium polyacrylate serving as a hydrophilic polymer was obtained. The structure of the resultant potassium polyacrylate was analyzed by NMR (AVANCE III-600 with Cryo Probe, manufactured by Bruker Biospin).

The results are shown in Table 1.

### [Example 6]

5 kg of the acrylic pressure-sensitive adhesive (A) obtained in Production Example 1 was prepared. While the acrylic pressure-sensitive adhesive (A) was loaded in portions into a planetary mixer (PLM-15, manufactured by Inoue Mfg., Inc.) serving as a reaction device and 2.5 kg of 1-butanol serving as a viscosity modifier was loaded in portions thereinto, stirring was continued, and the contents were mixed until the mixture became uniform. Thus, the acrylic pressure-sensitive adhesive (A) was swollen with 1-butanol to become an acrylic pressure-sensitive adhesive in a swollen gel state.

Subsequently, 2.8 kg of pulverized potassium hydroxide (KOH, purity: 85%) was loaded, and stirring was continued for 3 hours while a stirring rotation speed was increased.

When the reaction was advanced, vapor and a liquid were generated in the reaction device, and the generated vapor and liquid were recovered in a solvent recovery device (CA103, manufactured by Kobex Co., Ltd.) cooled with chiller water at 15°C under reduced pressure by reducing a pressure in the reaction device. The weight of the recovered liquid was 2.5 kg. The structure of a main component of the recovered liquid obtained by the recovery under reduced pressure was identified by GC/MS analysis. As a result, the main component was able to be identified as 1-butanol.

After the completion of the reaction, a solid substance remained in the reaction device. The solid substance was pulverized with the planetary mixer as it was, and a solvent containing ethyl acetate and methanol at a volume ratio of 1/1 was added thereto. The resultant was stirred to be washed, and the resultant solid substance was dried at 130°C for 2 hours. As a result, potassium polyacrylate serving as a hydrophilic polymer was obtained. The structure of the resultant potassium polyacrylate was analyzed by NMR (AVANCE III-600 with Cryo Probe, manufactured by Bruker Biospin).

The results are shown in Table 1.

### [Comparative Example 1]

Potassium hydroxide was dissolved in methanol so that the entire potassium hydroxide (KOH) concentration became 1.3 wt%. Then, cyclopentyl methyl ether (CPME) and methanol (MeOH) were mixed so as to achieve a volume ratio of 7/3 to prepare a treatment liquid.

3 kg of the acrylic pressure-sensitive adhesive (A) obtained in Production Example 1 was prepared. The acrylic pressure-sensitive adhesive (A) and 60 kg of the prepared treatment liquid were loaded into a 100 kg polymerization kettle (helical ribbon-type stirring blade), and stirring was performed for 10 hours so that an inner bath temperature became 50°C.

When the reaction was advanced, the acrylic pressure-sensitive adhesive (A) was once dissolved in the reaction liquid, and was then modified to hydrophilic to be precipitated. In addition, the generated alcohol was dissolved in the solution. The structure of a main component of the generated alcohol was identified by NMR (AVANCE III-600 with Cryo Probe, manufactured by Bruker Biospin). As a result, the main component was able to be identified as 1-butanol.

The precipitate was washed with ethyl acetate, and was then dried at 130°C for 2 hours. As a result, polyacrylic acid serving as a hydrophilic polymer was obtained. The structure of the resultant polyacrylic acid was analyzed by NMR (AVANCE III-600 with Cryo Probe, manufactured by Bruker Biospin).

The results are shown in Table 1.

### [Comparative Example 2]

Potassium hydroxide was dissolved in 1-butanol so that the entire potassium hydroxide (KOH) concentration became 3.0 wt%. Then, methylcyclohexane (MCH) and 1-butanol (BuOH) were mixed so as to achieve a volume ratio of 7/3 to prepare a treatment liquid.

2 g of the acrylic pressure-sensitive adhesive (A) obtained in Production Example 1 and 20 g of the prepared treatment liquid were loaded into a 50 cc screw tube, and were stirred for 8 hours together with a stirring bar so that an inner bath temperature became 40°C.

When the reaction was advanced, the acrylic pressure-sensitive adhesive (A) was once dissolved in the reaction liquid, and was then modified to hydrophilic to be precipitated. In addition, the generated alcohol was dissolved in the solution. The structure of a main component of the generated alcohol was identified by NMR (AVANCE III-600 with Cryo Probe, manufactured by Bruker Biospin). As a result, the main component was able to be identified as 1-butanol.

The precipitate was washed with ethyl acetate, and was then dried at 130°C for 2 hours. As a result, polyacrylic acid serving as a hydrophilic polymer was obtained. The structure of the resultant polyacrylic acid was analyzed by NMR (AVANCE III-600 with Cryo Probe, manufactured by Bruker Biospin).

The results are shown in Table 1.

### [Comparative Example 3]

2 g of the acrylic pressure-sensitive adhesive (A) obtained in Production Example 1 was loaded into a 50 cc screw tube. Further, 0.6 g of pulverized potassium hydroxide (KOH) was added.

The screw tube was sealed and then left as it was under a laboratory environment for 24 hours.

No liquid was generated, and no change was observed in the state of the acrylic pressure-sensitive adhesive (A). The structure of the solid content in the screw tube was analyzed by NMR (AVANCE III-600 with Cryo Probe, manufactured by Bruker Biospin).

The results are shown in Table 1.

**Table 1**

| | Acrylic pressu resensiti ve adhesi ve | Alkalin e compo und | Use of treatm ent liquid | Solven t in treatm ent liquid | Treatm ent liquid/a crylic pressu resensiti ve adhesi ve (weigh t ratio) | Organi c solvent (I) for swellin 9 acrylic pressu resensiti ve adhesi ve | Organi c solvent (l)/acry lic pressu resensiti ve adhesi ve (weigh t ratio) | Alkali conce ntratio n [wt%] | Means for stirring and kneadi ng | Reacti on time [hour(s )] | Kind of recove red alcohol | Carbo xylic acid conver sion rate [%] |
|---|---|---|---|---|---|---|---|---|---|---|---|---|
| Examp le 1 | (A) | KOH | None | None | - | None | - | Solid | Planet ary mixer | 0.5 | 1-Butano I | 95 |
| Examp le 2 | (A) | KOH | None | None | - | None | - | Solid | Knead er | 0.5 | 1-Butano l | 96 |
| Examp le 3 | (A) | NaOH | None | None | - | None | - | Solid | Trimix | 0.9 | 1-Butano l | 96 |
| Examp le 4 | (A) | KOH | None | None | - | None | - | Solid | Trimix | 0.5 | 1-Butano l | 96 |
| Examp le 5 | (B) | KOH | None | None | - | None | - | Solid | Trimix | 5 | Ethano I and 2-ethylh exanol | 95 |
| Examp le 6 | (A) | KOH | None | None | - | BuOH | 0.5 | Solid | Planet ary mixer | 3 | 1-Butano l | 95 |
| Comp arative Examp le 1 | (A) | KOH | Used | CPME /MeO H=7/3 | 20 | None | - | 1.3 | Helical ribbon-type stirring blade | 10 | 1-Butano l | 54 |
| Comp arative Examp le 2 | (A) | KOH | Used | MCH/ BuOH =7/3 | 10 | None | - | 3.0 | Stirrer | 8 | 1-Butano l | 57 |
| Comp arative Examp le 3 | (A) | KOH | None | None | - | None | - | Solid | None | No reactio n | None | 0 |

It was found from the results of Examples and Comparative Examples that the methods according to the embodiment of the present invention provided a hydrophilic polymer and an alcohol having high purity in a high yield in an extremely short reaction time by a solid phase reaction between an acrylic pressure-sensitive adhesive and an alkaline compound, without substantially using a treatment liquid such as a solvent. In addition, the carboxylic acid conversion rate of the obtained hydrophilic polymer was also extremely high.

### Industrial Applicability

The method of producing a hydrophilic polymer and the method of producing an alcohol according to the embodiments of the present invention can be suitably utilized in a technology of recycling, for example, a material of a pressure-sensitive adhesive tape.

## Claims

1. A method of producing a hydrophilic polymer from an acrylic pressure-sensitive adhesive, the method comprising a reaction step of subjecting the acrylic pressure-sensitive adhesive and an alkaline compound to a solid phase reaction.

2. The method of producing a hydrophilic polymer according to claim 1, wherein the solid phase reaction is performed by kneading the acrylic pressure-sensitive adhesive and the alkaline compound while applying shear force.

3. The method of producing a hydrophilic polymer according to claim 1, wherein an alcohol is generated through the reaction step.

4. The method of producing a hydrophilic polymer according to claim 3, further comprising an alcohol recovery step of recovering the alcohol generated through the reaction step.

5. The method of producing a hydrophilic polymer according to claim 4, wherein the alcohol recovery step includes recovering the alcohol generated through the reaction step under reduced pressure.

6. The method of producing a hydrophilic polymer according to claim 1, further comprising an impurity separation step of separating an impurity.

7. The method of producing a hydrophilic polymer according to claim 1, further comprising a drying step of drying the hydrophilic polymer.

8. The method of producing a hydrophilic polymer according to claim 1, wherein the acrylic pressure-sensitive adhesive is an acrylic pressure-sensitive adhesive in a swollen gel state containing an organic solvent.

9. The method of producing a hydrophilic polymer according to claim 8, wherein a content of the organic solvent in the acrylic pressure-sensitive adhesive in a swollen gel state is 50 wt% or less.

10. A method of producing an alcohol from an acrylic pressure-sensitive adhesive, the method comprising a reaction step of subjecting the acrylic pressure-sensitive adhesive and an alkaline compound to a solid phase reaction.

11. The method of producing an alcohol according to claim 10, wherein the solid phase reaction is performed by kneading the acrylic pressure-sensitive adhesive and the alkaline compound while applying shear force.

12. The method of producing an alcohol according to claim 10, wherein the acrylic pressure-sensitive adhesive is an acrylic pressure-sensitive adhesive in a swollen gel state containing an organic solvent.

13. The method of producing an alcohol according to claim 12, wherein a content of the organic solvent in the acrylic pressure-sensitive adhesive in a swollen gel state is 50 wt% or less.
